# EUROPEAN PATENT APPLICATION

(11) **EP 2 243 480 A1**
(43) Date of publication of application: **27.10.2010**
(21) Application number: 10006355.1
(22) Date of filing: 27.08.2004
(51) Int. Cl.: A61K 31/282, A61P 35/00

(54) **Pharmaceutical formulations comprising oxaliplatin and an acid.**

(30) Priority: 28.08.2003 AU 2003904627
(62) Divisional of application: 04761205.6
(71) Applicant: Mayne Pharma Pty Ltd, Melbourne VIC 3004 (AU)
(72) Inventor: Whittaker, Darryl Vanstone, Vermont Victoria 3133 (AU); Liu, Aikun Julie, Endeavour Hills Victoria 3802 (AU)
(74) Representative: Baldock, Sharon Claire

(57) **Abstract**

There is provided a pharmaceutical liquid formulation of oxaliplatin for parenteral administration, said formulation comprising (i) oxaliplatin; (ii) water; and (iii) an additive selected from the group consisting of tartaric acid, a salt of tartaric acid, a pharmaceutically acceptable derivative of tartaric acid and mixtures thereof; wherein the additive is at a concentration of at least 0.01 mM. Methods of preparing the formulation are disclosed. There is further provided the use of the formulation in the preparation of a medicament for the treatment of cancer and a method for treating cancer which comprises administering a therapeutic amount of a pharmaceutical formulation.

## Description

### FIELD OF THE INVENTION

The invention relates to formulations containing oxaliplatin.

### BACKGROUND OF THE INVENTION

Oxaliplatin is an anticancer agent. Oxaliplatin (CAS 61825-94-3), also known as L-OHP, is a third generation platinum complex. The term "oxaliplatin" as used herein includes cis-oxalato(trans-/-1,2-diaminocyclohexanc) platinum(II), its optic enantiomer cis-oxalato(trans-*d*-1,2-diaminocyclohexane) platinum(II), and any mixture thereof.

Oxaliplatin is currently approved and marketed for second-line treatment of colorectal cancer. Oxaliplatin is available in a lyophilised form (20 mg, 50 mg or 100 mg vials). Just prior to administration, the lyophilised powder is reconstituted using water for injection or 5% glucose injection solution to provide a solution containing 5 mg/ml oxaliplatin. Typically, the reconstituted solution is then further diluted in 250-500 mL of 5% glucose injection solution. The diluted oxaliplatin solution is then infused either by peripheral vein or central venous line over 2 to 6 hours.

Lyophilized oxaliplatin has some disadvantages as a pharmaceutical form. The manufacturing process for a lyophilised dosage form is complicated and expensive. For example, the risk of sterility failure during manufacture of freeze dried forms is generally higher than is the case for liquid solutions. In addition, the reconstitution of freeze dried preparations requires both skill and care as it involves several risks, *inter alia,* incomplete dissolution of the powder, contamination through handling a highly toxic substance as a powder or cake, and maintaining the sterility of both the vial and the infusion solution during reconstitution and transfer to the infusion bag. Thus, to administer a lyophilized drug, multiple handling of the drug is required - the lyophilised oxaliplatin is first reconstituted, then diluted with a 5% glucose solution and then administered by intravenous infusion.

Further, following reconstitution, oxaliplatin is prone to instability, particularly in solutions containing certain nucleophilic agents. For example, some reconstitution solutions containing chloride ions, such as 0.9% sodium chloride solution, are commonly used in hospitals. The mistaken use of such a reconstitution solution in the case of the lyophilized form of oxaliplatin has the serious consequence of rapidly decomposing the oxaliplatinum metal complex, forming a precipitate (dichtoro-diammocyclohexane-platinum derivative) with NaCl.

As a consequence of the limitations described above, several stabilised aqueous ready-to-use (RTU) liquid oxaliplatin preparations have been proposed:
1. US 5,716,988 and AU 731981 disclose a pharmaceutical formulation consisting of a 1 to 5 mg/mL solution of oxaliplatin in water having a pH range of 4.5 to 6. However, subsequently, WO 99/43355 and US 6,476,068 report that simple aqueous solutions of oxaliplatin prepared according to the methods taught in this specification are insufficiently stable.
2. WO 99/43355 and US 6,306,902 disclose an oxaliplatin solution formulation containing 1 to 7 mg/ml oxaliplatin, a buffering agent and a pharmaceutically acceptable carrier. The preferred buffering agent (and only example) is oxalic acid or an alkali metal salt thereof.
3. WO 01/15691 discloses solutions of at least 7 mg/ml oxaliplatin containing a solvent containing a sufficient amount of at least one hydroxylated derivative selected from 1,2-propane-diol, glycerol, maltitol, sucrose and inositol. The specification states that these are the only suitable agents to use after consideration of several options. Further, if buffering agents are used, the specification teaches that the buffer should have an oxalic acid base.
4. US 6,476,068 discloses an oxaliplatin solution formulation comprising 0.1 to 10 mg/ml oxaliplatin, an effective stabilizing amount of the monocarboxylic acid, lactic acid, and a pharmaceutically acceptable carrier. The preferred concentration range of oxaliplatin is 2 to 5 mg/ml.
5. US Patent Application No. 20030109515 discloses an oxaliplatin solution formulation containing a stabilising amount of malonic acid. The examples are directed to formulations having an oxaliplatin, concentration of 2 mg/ml. In contrast to the teaching of this application, and as is discussed below, the present inventors have found that malonic acid destabilises oxaliplatin in solution.

Buffering agents are used in liquid pharmaceutical formulations to adjust the pH of the formulation and to maintain the formulation within a deaired pH range. As mentioned above, the dicarboxylic acid, oxalic acid, and its salts have been proposed as a buffering and stabilising agent for oxatiplatin. Oxalate ion is formed in aqueous solutions of oxaliplatin by hydrolysis, thus conceivably this reaction may be slowed (using Le Chatelier's principle) through purposeful addition of oxalate ion to solutions of oxaliplatin. However, oxalic acid has some disadvantages as a pharmaceutical buffering agent, notably It's toxicity. Oxalic acid is potentially nephrotoxic and also requires special handling precautions, which complicate and limit its use in pharmaceutical products.

There is a need for agents that can be used with oxaliplatin solutions as alternatives to the prior art buffering agents (oxalic acid, lactic acid and malonic acid) and which do not have the disadvantages associated with the use of oxalic acid.

Ideally, the alternative agents would not destabilise oxaliplatin in solution. In particular, it would be useful if the alternative agent improve the stability of oxaliplatin in aqueous formulations in a manner that minimises significant degradation of oxaliplatin and limits the formation of unwanted impurities such as diaquo DACH platinum and diaquo DACH platinum dimer.

Further, it would be preferable to limit the amount of unknown degradation products in the aqueous formulation Any unknown degradation product present in an amount exceeding the thresholds set in the guidelines of the ICH (International Conference on Harmonisation of Technical Requirements for Registration of Pharmaceuticals for Human Use) is required to be identified. This imposes significant requirements on the manufacturer of the formulation, as they are required to identify trace amounts of an unknown degradation product. In addition, the presence of unknown degradation products is an indication that there may be additional risks of toxidty and unknown side-effects as a consequence of the presence of these products. It is therefore of interest to a manufacturer of a formulation to avoid producing unknown degradation products.

Ideally, additional pharmaceutically acceptable buffering agents should be non-toxic and be preset in the smallest possible quantity. Furthermore, during manufacture they should be introduced in the safest and most convenient manner possible.

### SUMMARY OF THE INTENTION

In a first aspect the present invention provides a pharmaceutical liquid formulation of oxaliplatin for parenteral administration, said formulation comprising
(i) oxalipratin;
(ii) water; and
(iii) an acid
   wherein the acid is stabilising and is not malonic acid, lactic acid or oxalic acid.

In a second aspect the present invention provides a pharmaceutical liquid formulation of oxaliplatin for parenteral administration, said formulation comprising
(i) oxaliplatin;
(ii) water; and
(iii) an acid comprising at least 4 carbon atoms.

In a third aspect the present invention provides a pharmaceutical liquid formulation of oxaliplatin for parenteral administration, said formulation comprising
(i) oxaliplatin,
(ii) water; and
(iii) an additive selected from the group consisting of a pharmaceutically acceptable carboxylic acid, a salt of a pharmaceutically acceptable carboxylic acid, a pharmaceutically acceptable derivative of a pharmaceutically acceptable carboxylic acid and mixtures thereof; wherein the additive is at a concentration of at least 0.01 mM and wherein the acid is not malonic acid, lactic acid or oxalic acid.

in a fourth aspect, the present invention provides a pharmaceutical liquid formulation of oxaliplatin for parenteral administration, said formulation comprising
(i) oxaliplatin,
(ii) water; and
(iii) an additive selected from the group consisting of a pharmaceutically acceptable carboxylic acid, a salt of a pharmaceutically acceptable carboxylic acid, a pharmaceutically acceptable derivative of a pharmaceutically acceptable carboxylic acid and mixtures thereof;
   wherein the additive is at a concentration of at least 0.01 mM and wherein the carboxylic acid is of the formula:

   HO2C[C(R1)(R2)]nCO2H

   wherein n = 2 to 6; and R1 and R2 are each independently selected from the group consisting of H, OH, CO2H, halo and methyl.

In a fifth aspect, the present invention provides a pharmaceutical liquid formulation of oxaliplatin for parenteral administration, said formulation comprising
(i) oxaliplatin,
(ii) water; and
(iii) an additive selected from the group consisting of tartaric acid, a salt of tartaric acid, a pharmaceutically acceptable derivative of tartaric acid and mixtures thereof;
   wherein the additive is at a concentration of at least 0.01 mM.

In a sixth aspect, the present invention provides for the use of a pharmaceutical formulation according to the first to third aspects in the preparation of a medicament for the treatment of a cancer.

In a seventh aspect, the present invention provides a method for treating a cancer which comprises administering a pharmaceutical formulation according to the first to third aspects to a patient in need thereof.

In a eighth aspect, there is provided a method for preparing pharmaceutical formulations according to the first to third aspects, the method comprising the steps of:
(i) dissolving oxaliplatin in water to form a solution;
(ii) dissolving the additive in the solution;
(iii) optionally, adjusting the pH of the solution with a pharmaceutically acceptable base.

In a ninth aspect, the present invention provides a pharmaceutical liquid formulation of oxaliplatin for parenteral administration, said formulation comprising
(i) about 5 mg/ml of oxaliplatin,
(ii) water, and
(iii) an additive consisting of tartaric acid and the sodium salt of tartaric acid,
   wherein the concentration of the additive is about 0.2 mM and wherein the pH of the solution is from about 4.7 to about 5.5.

In an tenth aspect, the present invention provides for the use of a pharmaceutical formulation according to the ninth aspect in the preparation of a medicament for the treatment of a cancer.

In a eleventh aspect, the present invention provides a method for treating a cancer which comprises administering a pharmaceutical formulation according to the seventh aspect to a patient in need thereof.

In a twelfth aspect, there is provided a method for preparing a pharmaceutical formulation, the method comprising the steps of:
(i) dissolving oxaliplatin in water to form a solution;
(ii) dissolving tartaric acid in the solution;
(iii) adjusting the pH of the solution with sodium hydroxide such that it is in the range of from 4.7 to 5.5
   wherein the concentration of oxaliplatin is about 5 mg/ml and the concentration of tartaric acid is about 0.2 mM.

### BRIEF DESCRIPTION OF THE FIGURES

**Figure 1(a)** is a chromatogram showing stability of a solution of oxaliplatin in water stored at 40°C for 12 weeks.
**Figure 1(b)** is a chromatogram showing stability of a solution of oxaliplatin and tartaric acid in water stored at 40ºC for 12 weeks.
**Figure 1(c)** is a chromatogram showing stability of a solution of oxaliplatin, tartaric acid and sodium tartrate in water stored at 40°C for 12 weeks.
**Figure 1(d)** is a chromatogram showing stability of solution of oxaliplatin and succinic acid disodium salt in water stored at 40°C for 12 weeks.
**Figure 1(e)** is a chromatogram showing stability of a solution of oxaliplatin, maleic acid and sodium hydroxide in water stored at 40°C for 12 weeks.
**Figure 2(a)** is a chromatogram showing stability of a solution of oxaliplatln in water stored at 40°C for 8 weeks.
**Figure 2(b)** is a chromatogram showing stability of a solution of oxaliplatin and tartaric acid in water stored at 40°C for 8 weeks.
**Figure 2(c)** is a chromatogram showing stability of a solution of oxaliplatin, tartaric acid and sodium tartrate in water stored at 40°C for 8 weeks.
**Figure 2(d)** is a chromatogram showing stability of a solution of oxaliplatin, tartaric acid and sodium tartrate in water at 40°C for 8 weeks, the ratio of tartrate to tartaric acid being greater than for the solution of figure 2(c).

### DETAILED DESCRIPTION OF THE INVENTION

In a first aspect, the present invention provides a pharmaceutical liquid formulation of oxaliplatin for parenteral administration, said formulation comprising
(i) oxaliplatin;
(ii) water; and
(iii) an acid
   wherein the acid is stabilising and is not malonic acid, lactic acid or oxalic acid.

It is preferred that the acid is a carboxylic acid, preferably a dicarboxylic acid.

In a preferred embodiment the acid is selected from the group consisting of citric acid, maleic acid, saccharic acid, succinic acid, malic acid, tartaric acid and mixtures thereof. It is preferred that the acid is malic acid, succinic acid, tartaric acid and mixtures thereof and is most preferably tartaric acid.

In a second aspect, the present invention provides a pharmaceutical liquid formulation of oxaliplatin for parenteral administration, said formulation comprising
(i) oxaliplatin;
(ii) water; and
(iii) an acid comprising at least 4 carbon atoms.

It is preferred that the acid is a dicarboxylic acid and preferably comprises 4 to 10 carbon atoms, more preferably 4 to 6 carbon atoms.

In a further embodiment the acid comprises 1 or 2 hydroxyl groups.

In a preferred embodiment the acid is selected from the group consisting of citric acid, maleic acid, saccharic acid, succinic acid, malic acid, tartaric acid and mixtures thereof. It is preferred that the acid is malic acid, succinic acid, tartaric acid and mixtures thereof and is most preferably tartaric acid.

In a still further preferred embodiment of the first and second aspects of the invention the acid is at a concentration of at least 0.01 mM.

In a third aspect, the present invention provides a pharmaceutical liquid formulation of oxaliplatin for parenteral administration, said formulation comprising
(i) oxaliplatin,
(ii) water; and
(iii) an additive selected from the group consisting of a pharmaceutically acceptable carboxylic acids, a salt of a pharmaceutically acceptable carboxylic acid, a pharmaceutically acceptable derivative of a pharmaceutically acceptable carboxylic acid and mixtures thereof;
   wherein the additive is at a concentration of at least 0.01 mM and wherein the acid is not malonic acid, lactic acid or oxalic acid.

Preferably, the add is a dicarboxylic acid.

Preferably, the acid is selected from the group consisting of citric acid, maleic acid, saccharic acid, succinic acid, malic acid, tartaric acid and mixtures thereof.

In a fourth aspect, there is provided a pharmaceutical liquid formulation of oxaliplatin for parenteral administration, said formulation comprising
(i) oxaliplatin,
(ii) water; and
(iii) an additive selected from the group consisting of a pharmaceutically acceptable carboxylic acid, a salt of a pharmaceutically acceptable carboxylic acid, a pharmaceutically acceptable derivative of a pharmaceutically acceptable carboxylic acid and mixtures thereof;
   wherein the additive is at a concentration of at least 0.01 mM and wherein the carboxylic acid is of the formula:

   HO2C[CR1)(R2)]nCO2H
wherein n = 2 to 6; and R1 and R2 are each independently selected from the group consisting of H, OH, CO2H, halo and methyL

Pharmaceutically acceptable acids include glutaric acid, citric acid, malic acid, succinic acid, tartaric acid and mixtures thereof. Preferably the pharmaceutically acceptable carboxylic acid is selected from the group consisting of malic acid, succinic acid, tartaric acid and mixtures thereof. More preferably, the pharmaceutically acceptable carboxylic acid is tartaric acid.

Preferably n = 2 to 4; more preferably n = 2.

Tartaric acid (HOOCCH(OH)CH(OH)COOH) is used in food and pharmaceutical formulation as an acidulant, sequestering agent, or antioxidant synergist. In pharmaceutical formulations, it is widely used in combination with bicarbonates, as the acid component of effervescent granules, powders, and tablets. It displays none of the toxicity associated with the use of oxalic acid as a stabilising agent, such as nephrotoxicity.

Succinic acid (HOOCCH₂CH₂COOH) is used as a food additive and in detergents and cosmetics. It can be found naturally occurring in animal tissues, and in vegetables or fruit.

Malic acid (HOOCCH(OH)CH2COOH) is used as a flavouring agent, flavour enhancer and acidulant in foods. It is found naturally in apples and many other fruits.

Citric acid is (2-hydroncy-1,2,3-propane-tricarboxylic acid) is widely distributed in plants and in animal tissues and fluids.

Maleic acid has the formula HOOCCH=CHCOOH.

Saccharic add has the formula HOOC[CHOH]₄COOH and is derived from starch.

In a fifth aspect, the present invention provides a pharmaceutical liquid formulation of oxaliplatin for parenteral administration, said formulation comprising:
(i) oxaliplatin,
(ii) water; and
(iii) an additive selected from the group consisting of tartaric acid, a salt of tartaric acid, a pharmaceutically acceptable derivative of tartaric acid and mixtures thereof;
   wherein the additive is at a concentration of at least 0.01 mM.

Many of the carboxylic acids of the present invention are found as isomers. For instance, tartaric acid has many isomeric forms. The present invention contemplates the use of any of the isomer of the carboxylic acid used as an additive. For instance, where the carboxylic acid is tartaric acid, the tartaric acid may be elected from any of the isomers of tartaric acid including the group consisting of (+)-tartaric acid, (-)-tartaric acid, mesotartaric acid and mixtures thereof. Preferably, the tartaric acid is (+)-tartaric acid.

Where the additive is a mixture of a pharmaceutically acceptable carboxylic acid and a salt of a pharmaceutically acceptable carboxylic acid, the concentration of the additive is the sum of the concentrations of the carboxylic acid and the salt. Preferably, where the additive is a mixture of a pharmaceutically acceptable carboxylic acid and a salt of a pharmaceutically acceptable carboxylic acid, the salt is the conjugate base of the carboxylic acid so as to form a buffer solution.

When the additive includes a salt of a pharmaceutically acceptable carboxylic acid, the salt of may be formed in situ by the addition of a pharmaceutically acceptable base to an acid solution. Alternatively, the salt may be added directly to the formulation.

Preferably, the concentration of the additive in the formulations of the first to third aspects of the invention is from about 0.01 mM to about 2.0 mM, more preferably from about 0.1 mM to about 1.0 mM, even more preferably from about 0.1 mM to about 0.6 mM, yet more preferably from about 0.2 mM to about 0.6 mM.

Preferably, when the additive comprises a salt of a pharmaceutically acceptable acid the salt is a sodium salt.

Pharmaceutically acceptable derivatives of carboxylic acids include but are not limited to such derivatives as esters, amides, carbonates and carbamates of the acid.

The amount of oxaliplatin present in a pharmaceutical formulation according to the invention is preferably up to about 15 mg/ml, preferably about up to about 7 mg/ml. Preferably the amount of oxaliplatin is in the range of from 1 to 5 mg/ml and most preferably is about 5 mg/ml.

As will be understood, the additive should be used at a concentration which does not destabilise the oxatiplatin and preferably aids stability of the oxaliplatin. The desired stability of oxaliplatin will depend on the intended shelf life of the pharmaceutical formulation and the manipulation prior to administration. More specifically, a stable aqueous oxaliplatin formulation is one in which there will be no significant change in oxaliplatin potency at the specified storage condition. The criteria for "significant change" are as defined in the International Conference on Harmonisation (ICH) Guideline: Stability Testing of New Drug Substances and Products Q1A (R2) Thus in the case of injectable RTU oxaliplatin solution, potency of oxaliplatin should be at least 95% of initial content, and solution remains clear, colourless and free of precipitation for a pharmaceutically acceptable duration of time.

Preferably, the additive is at a concentration sufficient to buffer the formulation at a pH in the range of from about 3 to about 8, more preferably about 4 to about 7, even more preferably about 5.

As is known to a person skilled in the art a buffering system is a mixture of an acid with it conjugate base in a solution, the mixtures being formulated so as to maintain the pH at a desired level. As defined herein, "buffering agent" refers to an acid or a base which may form a component of a buffering system whether or not the acid or base is associated with its conjugate base or conjugate add, respectively.

Preferably the pharmaceutical formulation of the invention is provided in a sterile, sealed container. For example, a neutral glass of type I and a stopper. Examples of the stopper include those made of an elastomer based on halogenated butyls, possibly coated with a fluorinated polymer.

In a sixth aspect of the present invention there is provided the use of the formulations of any one of the first to third aspects in the preparation of a medicament for the treatment of a cancer.

In a seventh aspect of the present invention there is provided a method for treating a cancer which comprises administering a pharmaceutical formulation according to the any one of the first to third aspects to a patient in need thereof.

The cancer can be any cancer that is amenable to treatment by oxaliplatin, either alone or in combination with other chemotherapeutic agents, and includes colorectal cancer.

The term "treating" as used herein, unless otherwise indicated, means reversing, alleviating, inhibiting the progress of, or preventing the disorder or condition to which such term applies, or one or more symptoms of such disorder or condition. The term "treatment", as used herein, refers to the act of treating, as "treating" is defined immediately above.

In the above methods, the effective dosage of oxaliplatin to be administered to a patient ranges from about 10 mg/m² to about 250 mg/m², more preferably from about 30 mg/m² to about 180 mg/m² and most preferably is about 85 mg/m². However, it will be understood that the therapeutic dosage administered will be determined by the physician in the light of the relevant circumstances including the severity of the condition to be treated and the chosen route of administration. Therefore, the above dosage ranges are not intended to limit the scope of the invention in any way. Administration of oxaliplatin will typically be according to best practice known to those skilled in the art at the time of administration.

The present invention also provides methods of preparing the formulations of the present invention. Accordingly, in a further aspect there is provided a method for preparing a pharmaceutical formulation, the method comprising the steps of:
(i) dissolving oxaliplatin in water to form a solution;
(ii) dissolving in the solution an additive selected from the group consisting of a pharmaceutically acceptable carboxylic acid, a salt of a pharmaceutically acceptable carboxylic acid, a pharmaceutically acceptable derivative of a pharmaceutically acceptable carboxylic acid and mixtures thereof;
(iii) optionally, adjusting the pH of the solution with a pharmaceutically acceptable base wherein the acid is not malonic acid, lactic acid or oxalic acid.

In another aspect the present invention provides a method for preparing a pharmaceutical formulation, the method comprising the steps of:
(i) dissolving oxaliplatin in water to form a solution;
(ii) dissolving in the solution an additive selected from the group consisting of a pharmaceutically acceptable carboxylic acid, a salt of a pharmaceutically acceptable carboxylic acid, a pharmaceutically acceptable derivative of a pharmaceutically acceptable carboxylic acid and mixtures thereof;
(iii) optionally, adjusting the pH of the solution with a pharmaceutically acceptable base wherein the carboxylic acid is of the formula:

   HO2C[C(R₁)(R₂)]nCO2H

   wherein n = 2 to 6; and R1 and R2 are each independently selected from the group consisting of H, OH, CO2H halo and methyl.

Preferably, n = 2 to 4. More preferably, n = 2.

In yet another aspect the present invention provides a method for preparing a pharmaceutical formulation, the method comprising the steps of:
(i) dissolving oxaliplatin in water to form a solution,
(ii) dissolving in the solution an additive selected from the group consisting of a tartaric acid, a salt of tartaric acid, a pharmaceutically acceptable derivative of a pharmaceutically acceptable tartaric acid and mixtures thereof;
(iii) optionally, adjusting the pH of the solution with a pharmaceutically acceptable base. pH adjustment may be carried out with any pharmaceutically acceptable base. Preferably the pharmaceutically acceptable base is a sodium hydroxide (NaOH) solution.

In a further aspect, the present invention provides a pharmaceutical liquid formulation of oxaliplatin for parenteral administration, said formulation comprising
(i) about 5 mg/ml of oxaliplatin,
(ii) water, and
(iii) an additive consisting of tartaric acid and the sodium salt of tartaric acid,
   wherein the concentration of the additive is about 0.2 mM and wherein the pH of the solution is from about 4.7 to about 5.5.

The present invention also provides for the use of the formulations of the present invention in the preparation of a medicament for the treatment of a cancer and in the treatment of cancer in patients.

In a still further aspect, the present invention provides a method for preparing a pharmaceutical formulation, the method comprising the steps of:
(i) dissolving oxaliplatin in water to form a solution;
(ii) dissolving tartaric acid in the solution;
(iii) adjusting the pH of the solution with sodium hydroxide such that it is in the range of from 4.7 to 5.3
   wherein the concentration of oxaliplatin is about 5 mg/ml and the concentration of tartaric acid is about 0.2 mM.

In order that the nature of the present invention may be more clearly understood, preferred forms thereof will now be described with reference to the following non-limiting examples.

### EXPERIMENTAL

### Measurement of Stability of Oxaliplatin formulations

The stability of an oxaliplatin formulation over a period of time can be measured by a number of complementary methods. Visual appearance and stability of the pH of the formulation are important indicators and these can be measured by technique well known to those skilled in the art.

Stability can also be measured by high pressure liquid chromatography (HPLC) techniques. HPLC is a technique that is widely used and well known in the art. HPLC can be used to measure the potency of the oxaliplatin where potency is defined as a percentage of the initial concentration of oxaliplatin. HPLC can also be used to measure the relative proportions of known and unknown degradants in an oxaliplatin solution.

Known degradation products of oxaliplatin include:
- (trans-*l*-1,2diaminocyclohexane)trans-dihydroxo(oxalato) platinum (IV). This a oxidative degradation product of oxaliplatin. This degradation product has been designated as Impurity C in the Examples.
- (SP-4-2)-diaqua-[(1R,2R)-cyclohexane-1,2-diamine-kN,kN']platinum, or diaqua DACH platinum. This is a hydrolysis degradation product of oxaliplatin. This degradation product has been designated as Impurity B in the Examples.
- (SP-4-2)-di-µ-oxobis[(1R,2R)-cyclohexane-1,2-diamine-kN,kN']platinum, or diaqua DACH platinum dimer. This is a degradation product resulting from further reaction of Impurity B. This degradation product has been designated as Dimer in the Examples.

R,S-oxaliplatin is an isomeric form of oxaliplatin which is found at low levels as an impurity in oxaliplatin (ie cis-oxalato(trans-*l*-1,2-diaminocyclohexane) platinum(II)).

### Overview of the Examples

Example 1 details an initial trial of oxaliplatin formulations using a number of agents over a pH range from 3 to 7 in which the ability of tartaric acid, maleic acid, succinic and malic acids to stabilise oxaliplatin was compared to a control. Of the acids tested, tartaric acid was found to give the most stable oxaliplatin solutions and it was subsequently tested across a wide pH and concentration range as reported in Example 2. This study confirmed the advantages of tartaric acid and also indicated that there was a preferred concentration range for improved stability. A further study, reported in Example 3, then reviewed a number of other acids at a set concentration (0.3 mM) as buffering agents in solutions of oxaliplatin. This showed that formulations containing malic and succinic acid had impurity levels which were comparable to the tartaric acid formulation. Contrary to the teaching of the prior art, the malonic acid formulation contained a surprisingly and unacceptably high level of impurities relative to the tartaric acid formulation. Solutions containing citric, maleic and saccharic adds also showed reasonably low levels of impurity. The stabilisers of the prior art, oxalic acid and lactic acid, also displayed reasonably low levels of impurity which is unsurprising in the case of oxalic acid due to the operation of Le Chatelier's principle. Example 4 provides details of a preferred formulation of an aqueous solution of oxaliplatin and tartaric acid.

### Example 1

The stability of an array of oxaliplatin formulations in water for injection (WFI) having an oxaliplatin concentration of 5mg/ml was assessed. Potential buffering agents of oxaliplatin that were tested were tartaric acid, malic acid, Succinic acid and maleic acid. The pH of the formulations covered a range of values.

### Comparative Example 1(a)

### Preparation of the Control solution

WFI (water for injection) was added to a suitable glass vessel to about 80 % of the desired quantity of final volume and warmed to 45-50°C. While stirring and flushing with nitrogen, the desired quantity of oxaliplatin (calculated at 5 mg/mL at the final desired volume) was added and dissolved. The solution was then made up to the desired final volume with WFI.

### Example 1(b)

### Preparation of Dicarboxylic Acid Solutions

For the formulations described bellow, WFI was added to a suitable glass vessel to about 80% of the desired final volume and warmed to 45-50°C. While stirring and flushing with nitrogen, the desired quantity of oxaliplatin was added and dissolved. Thereafter the proposed stabilising dicarboxylic acid or its alkali salt was added to the oxaliplatin solution until completely dissolved. Where required, pH was adjusted through the addition of dilute NaOH solution. The solution so formed was made up to the final volume with WFI.

**Table 1 Oxaliplatin formulations containing maleic and malic acid based agents**

| **Formulation** | **Maleic pH 3** | **Maleic pH 5** | **Maleic pH 7** | **Malic pH 7** |
|---|---|---|---|---|
| **Oxaliplatin (mg)** | 5 mg | 5 mg | 5 mg | 5mg |
| **Malic acid disodium salt (mg)** | N/a | N/a | N/a | 1.5mg |
| **Maleic acid (mg)** | 0.03 mg | 0.08 mg | 0.71 mg | N/a |
| **HaOH 10N** | N/a | 0.067 µL | 15.9 µL | N/a |
| **WFI qs** | 1 mL | 1 mL | 1 mL | 1 mL |
| **Initial pH of the final formulation** | 3.50 | 5.38 | 7.16 | 6.92 |

**Table 2 Oxatiplatin formulations containing succinic add based agents**

| **Formulation** | **Succinic pH 3** | **Succinic pH 5** | **Succinic pH 7** |
|---|---|---|---|
| **Oxaliplatin 5 mg** | 6 mg | 5 mg | 5 mg |
| **Buccinic acid disodium salt** | N/a | 0.013 mg | 1.033 mg |
| **Sucoinic acid** | 0.13 mg | 0.003 | N/a |
| **WFI qs** | 1 mL | 1 mL | 1 mL |
| **Initial pH of the final formulation** | 3.57 | 6.03 | 6.91 |

**Table 3 Oxaliplatin formulations containing tartaric acid based agents**

| **Formulation** | **Tartaric pH 3** | **Tartaric pH 5** | **Tartaric pH 7** |
|---|---|---|---|
| **Oxaliplatin** | 1.5 mg | 5 mg | 5 mg |
| **NaOH 10N** | N/a | 0.033 µL | 0.067 µL |
| **Initial pH of the final formulation** | 3.56 | 4.8 | 7.08 |
| **Molarity of Tartaric Acid/Tartrate in Solution** | 2.9 × 10⁻⁴M | 2.2 × 10⁻⁴M | 3.1 × 10⁻⁴M |

The pH values used to designate the different formulations arc indications only and do not necessarily reflect the exact pH of each solution. The exact initial pH values are provided in the tables above.

### Example 1(c)

### Stability Study

In accordance with an accelerated stability protocol, the formulations were stored at 40°C with 75% relative humidity for 12 weeks.

The potency of the formulations was examined by high performance liquid chromatography (HPLC) at 4 week intervals over the 12 week period. Potency is defined as a percentage of the initial concentration of oxaliplatin. Most formulations maintained at least 95% potency over the 12 week period. The exception to this was Maleic pH 7 which had a potency of 41.9% after the 12 week period. Maleic acid could not therefore be considered as a viable stabiliser.

In respect of the use of malic acid as an stabiliser, the study of Malic pH 7 was terminated after 4 weeks due to significant precipitation and colour changes within the formulation. Accordingly, malic acid could not be considered as a viable stabiliser. As is discussed below this result may have been a consequence of the high concentrations of the malic acid disodium salt in the formulation.

Only very low levels of the oxidative degradation product Impurity C [(trans-*l*-1,2-diaminocyclohexano)trans-dihydroxo(oxalato) platinum (IV)] were detected in the formulations. This indicated that the formulations were substantially free of oxygen.

### Example 1(d)

### Study of the Degradation Products of Oxaliplatin at 12 weeks

Formulations Control, Tartaric pH 3, Tartaric pH7, Succinic pH 7 and Maleic pH 7 were analysed after 12 weeks at 40°C with 75% relative humidity for the presence of major degradation products of oxaliplatin [Impurity B( diaqua DACH platinum) and Dimer (diaqua DACH dimer)] using HPLC.

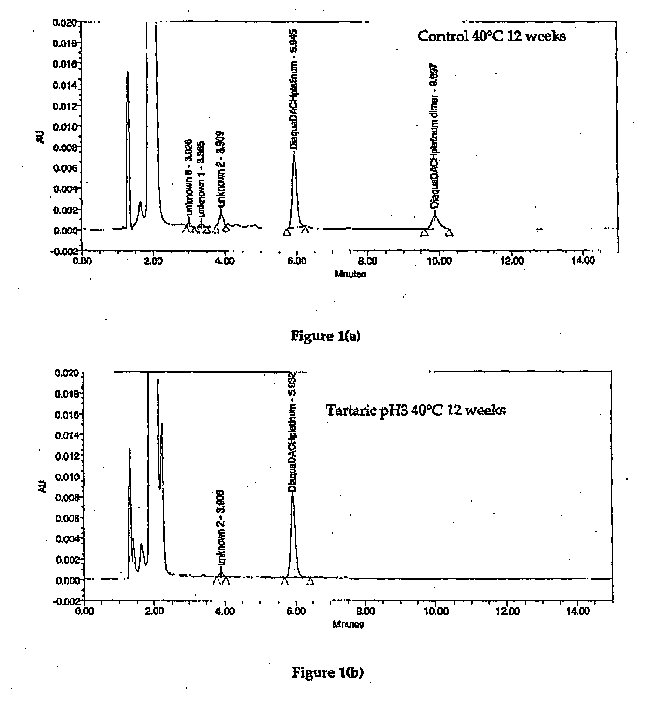

The chromatograms of the formulations are presented in Figures 1(a)-(e). The impurity peaks at above 0.01% are reported.

### Figure 1(a)

### Control 40°C 12 weeks

This system displays an impurity peak at 5.945 minutes corresponding to Impurity B (diaqua DACH platinum) and a further peak at 9.897 minutes corresponding to Dimer (diaqua DACH platinum dimer). A further three unknown impurity peaks are present. One is present at 3.909 minutes at a level of 0.03% and two at 3.026 and 3.386 minutes at 0.01%.

### Figure 1(b)

### Tartaric pH3 40°C 12 weeks

An impurity peak is present at 5.932 minutes which has been allocated to impurity B (diaqua DACH platinum). There is also present an impurity at 3.906 minutes. There is no impurity peak corresponding to Dimer (diaqua DACH platinum dimer).

### Figure 1(c)

### Tartaric pH7 40°C 12 weeks

This system displays an impurity peak is present at 5.931 minutes which corresponds to Impurity B (diaqua DACH platinum). There are also three unknown impurity peaks eluted at 3.027 minutes, 3.387 minutes and 3.906 minutes at the level of 0.01, 0.01 and 0.03% respectively. There is no impurity peak corresponding to Dimer (diaqua DACH platinum diner).

### Figure 1(d)

### Succinic pH7 40°C 12 weeks

This system displays a large number of unknown impurity peaks. These are present at 3.075 minutes, 3.581 minutes, 4.007 minutes, 4.164 minutes, 4.368 minutes , 6.512 minutes and 7.684 minutes. An impurity peak is also present at 5.956 minutes which corresponds to impurity B (diaqua DACH platinum).

### Figure 1(e)

### Maleic Acid pH7 40°C 12 weeks

The system displays a large number of unknown impurity peaks present at 2.587 minutes 2.751 minutes, 2.880 minutes, 3.042 minutes, 3.378 minutes, 3.599 minutes, 3.983 minutes, 4.203 minutes and 5.339 minutes.

It is clear from a visual comparison of the chromatograms that the tartaric acid stabilised formulations are far more stable than the maleic acid and succinic acid stabilised formulations. In addition, in comparison to the chromatogram of the control formulation, the formation of Dimer (diaqua DACH platinum dimer) is suppressed in the tartaric acid stabilised formulations. Further, at least in the case of the Tartaric pH 7 formulation, significantly less Impurity B (diaqua DACH platinum), the principle degradant, is formed. In addition, the tartaric acid stabilised formulations do not displays as many unknown impurity peaks as the control formulation.

### Example 1(e)

### Study of the Degradation Products of Oxaliplatin at 8 weeks

Formulations Control, Tartaric pH 3, Tartaric pH 5 and Tartaric pH7 were analysed after 8 weeks at 40°C with 75% relative humidity for the presence of degradation products of oxaliplatin using the HPLC protocol of Example 4.

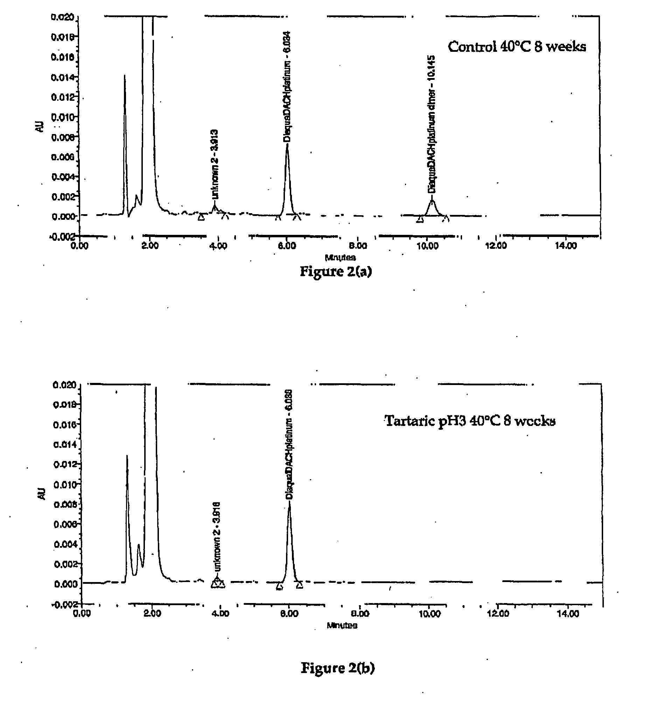

The chromatograms are presented in Figures 2(a)-(e)

### Figure 2(a)

### Control 40°C 8 weeks

This system displays an impurity peak at 6.304 minutes corresponding to Impurity B (diaqua DACH platinum) and further peak at 10.145 minutes corresponding to Dimer (diaqua DACH platinum dimer). An unknown impurity peak is present at 3.913 minutes.

### Figure 2(b)

### Tartaric pH 3 40°C 8 weeks

This system displays an impurity peak at 6.306 minutes corresponding to Impurity B (diaqua DACH platinum). There is no peak corresponding to the presence of Dimer (diaqua DACH platinum dimer). An unknown impurity peak is present at 3.916 minutes.

### Figure 2(c)

### Tartaric pH 5 40°C 8 weeks

This system displays an impurity peak at 6.306 minutes corresponding to impurity B (diaqua DACH platinum). There is no significant peak corresponding to the presence of Dimer (diaqua DACH platinum dimer). An unknown impurity peak is present at 3.911 minutes.

### Figure 2(d)

### Tartaric pH 7 40°C 8 weeks

This system displays an impurity peak at 6.306 minutes corresponding to impurity B (diaqua DACH platinum). There is no significant peak corresponding to the presence of Dimer (diaqua DACH platinum dimer). An unknown impurity peak is present at 3.913 minutes.

In comparison to the chromatogram of the control formulation, Dimer (diaqua DACH platinum dimer) formation is suppressed in the tartaric acid stabilised formulations.

### Summary

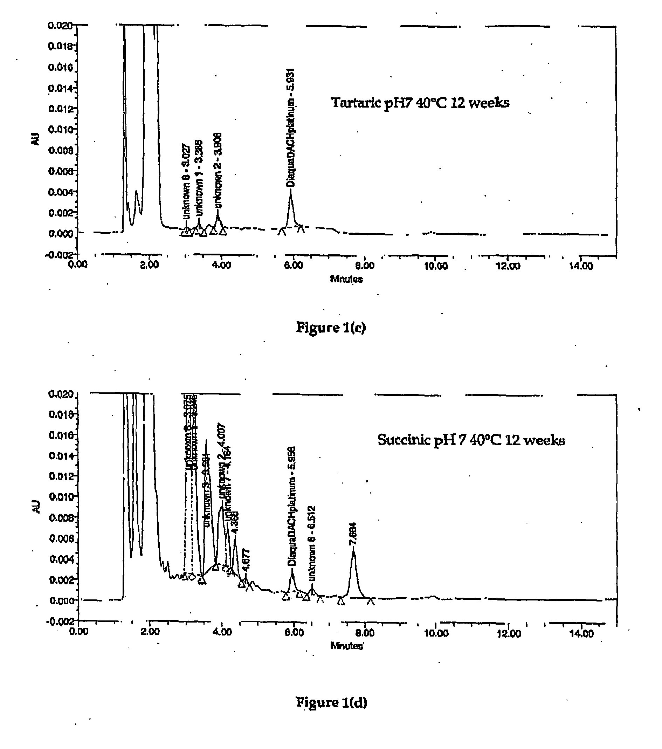

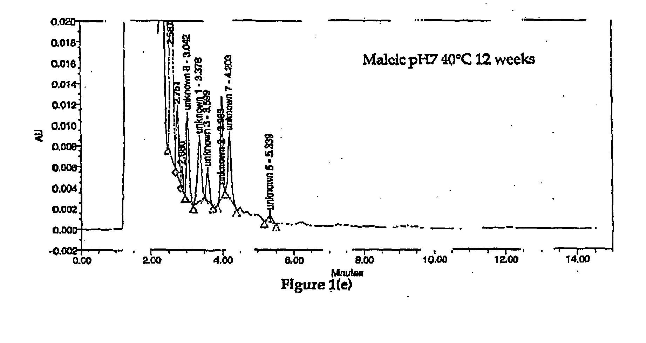

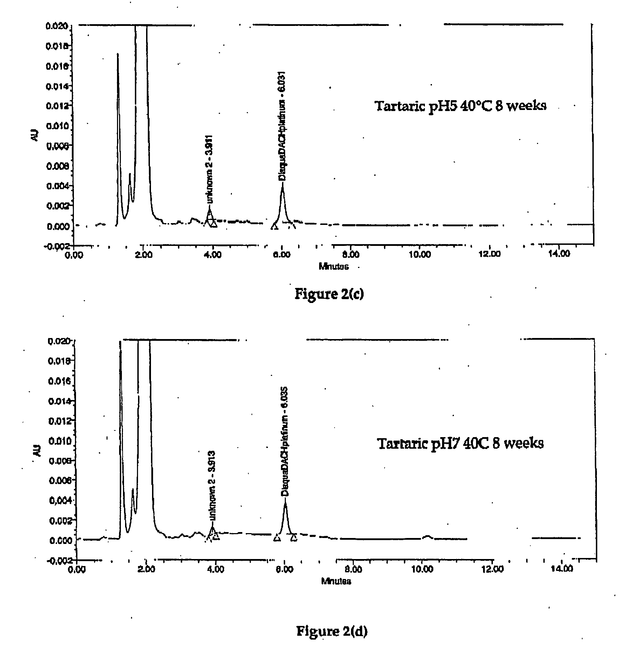

It is clear from a visual comparison of the chromatograms of Figures 1 and 2 that the tartaric acid stabilised foundations of oxaliplatin are far more stable than the maleic acid and succinic acid containing formulations. In addition, in comparison to the chromatogram of the control formulation, the formation of Dimmer (diaqua DACH platinum dimer) is suppressed in the tartaric acid stabilised formulations and, in some cases, significantly less Impurity B (diaqua DACH platinum), the principle degradant, is formed. Further, the tartaric acid stabilised formulations do not display as many unknown impurity peaks as the control formulation which is of importance in meeting the guide lines of the ICH and also in minimising any side effects due to the presence of unknown impurities. The increased stability of the oxaliplatin formulations applies across a range of pH values.

Although malic acid and succinic acid were considered as unsuitable agents following this experiment, later investigations, as detailed in Example 3, demonstrated that malic acid, succinic acid and maleic acid can be used with oxaliplatin. A possible reason for the initial finding that these formulations were unsuitable is because of the relatively high concentrations of certain of the succinic and malic acid formulations used in Example 1. For instance, the succinic pH 3 formulation has a concentration of succinic acid of about 1.10 mM and the succinic acid pH 7 formulation has a concentration of disodium salt of succinic acid of 6.38 mM. Similarly, the concentration of malic acid disodium salt in the malic pH 7 formulation is 8.42 mM. The concentration of maleic acid in the Maleic pH 7 formulation is about 6.1 mM. By contrast the tartaric acid concentrations of the formulations of this Example range from about 0.2 to about 0.3 mM.

### Example 2

### 2.1 Background

This Example was conducted to further investigate the effect of different amounts of tartaric acid and the effect of pH on the stability of oxaliplatin solution formulations. The tartaric acid formulations were compared to a control formulation of oxaliplatin in water and to a formulation of oxaliplatin in oxalic acid solution (according to US 6,306,902).

### 2.2 Preparation of formulations for analysis

### 2.2.1 Mixing procedure for the formulations

- Add about 80% mL of desired amount of WFI into a 2L mixing vessel and heat to 45-50°C, while stirring and flushing with nitrogen.
- Add oxaliplatin (total 7.5g) and mix until solution becomes clear.
- Adjust to the volume with WFI to 1500 mL.
- Divide the bulk solution to 100 mL each. Keep one 100 mL solution as the control.
- Add the required amount of tartaric acid solution 5% w/v or oxalic acid and NaOH (10N, 5N and/or 2N) according to the formulation details in Tables 5 and 6.
- Cap the final solution and keep in the fridge until filling.

### 2.2.2 Filling and capping

• Filter each formulation using a 0.2 µm syringe filter.
• Fill 2.0 mL of each formulation filled into a 2mL vial and cap. Tables 5 and 6 indicate the quantities of reagents added for each different formulation.

**Table 4 Formulation details for the oxaliplatin solutions containing tartaric acid of Example 2**

| **Item** | **Tartaric** (0.0003M) | | | | | **Tartaric** (0.0006M) | | **Tartaric** (0.0030M) | | **Tartaric** (0.0067M) | | **Tartaric** (0.0002M) | |
|---|---|---|---|---|---|---|---|---|---|---|---|---|---|
| Formulation | A2.5 | A4.0 | A5.0 | A7.0 | A8.5 | B4.0 | B7.0 | C4.0 | C7.0 | D4.0 | D7.0 | E4.0 | E7.0 |
| ID | | | | | | | | | | | | | |
| Oxaliplatin | 5 | 5 | 5 | 5 | 5 | 5 | 5 | 5 | 5 | 5 | 5 | 5 | 5 |
| (mg) | | | | | | | | | | | | | |
| Tartaric acid | 0.05 | 0.05 | 0.05 | 0.05 | 0.05 | 0.09 | 0.09 | 0.45 | 0.45 | 1.0 | 1.0 | 0.03 | 0.03 |
| (mg) | | | | | | | | | | | | | |
| WFI qs | 1 | 1 | 1 | 1 | 1 | 1 | 1 | 1 | 1 | 1 | 1 | 1 | 1 |
| (mL) | | | | | | | | | | | | | |
| Target pH | 2.5 | 4.0 | 5.5 | 7.0 | 8.5 | 4.0 | 7.0 | 4.0 | 7.0 | 4.0 | 7.0 | 4.0 | 7.0 |

| | | | | | | | | | | | | | |
|---|---|---|---|---|---|---|---|---|---|---|---|---|---|
| Note: Molecular weight of tartaric acid = 150.09 A = Formulation containing tartaric acid at 0.0045% (0.3 mM) B = Formulation containing tartaric acid at 0.009% (0.6 mM) C = Formulation containing tartaric acid at 0.045% (3 mM) D = Formulation containing tartaric acid at 0.1% (6.7 mM) E = Formulation containing tartaric acid at 0.003% (0.2 mM) | | | | | | | | | | | | | |

**Table 5: Quantity of oxaliplatin and excipients required to be used for the preparation of the oxaliplatin solutions containing tartaric acid of Example 2**

| **Item** | **Tartaric** (0.0003M) | | | | | **Tartaric** (0.0006M) | | **Tartaric** (0.0030M) | | Tartaric (0.0067M) | | **Tartaric** (0.0002M) | |
|---|---|---|---|---|---|---|---|---|---|---|---|---|---|
| Formulation ID | A2.5 | A4.0 | A5.0 | A7.0 | A8.5 | B4.0 | B7.0 | C4.0 | C7.0 | D4.0 | D7.0 | E4.0 | E7.0 |
| Oxaliplatin (mg) | 500 | 500 | 500 | 500 | 500 | 500 | 500 | 500 | 500 | 500 | 500 | 500 | 500 |
| Tartaric acid (mg) | 4.5 | 4.5 | 4.5 | 4.5 | 4.5 | 9 | 9 | 45 | 45 | 100 | 100 | 3 | 3 |
| Tartaric acid 5 %w/v (µL) | 90 | 90 | 90 | 90 | 90 | 180 | 180 | 900 | 900 | 2000 | 2000 | 60 | 60 |
| WFI qs (mL) | 100 | 100 | 100 | 100 | 100 | 100 | 100 | 100 | 100 | 100 | 100 | 100 | 100 |

**Table 6 Formulation details for the oxaliplatin control formulation and oxaliplatin solutions containing oxalic acid of Example 2.**

| Item | Control | Oxalic acid (0.001M) |
|---|---|---|
| Oxaliplatin (mg) | 5 | 5 |
| Oxalic acid (mg) | N/a | 0.126 |
| WFI qs (mL) | 1 | 1 |
| Target pH | 5.5 | 3.0 |

| | | |
|---|---|---|
| Note: In the initial screening, the pH 3 tartaric formulation was formulated at pH 3.78. | | |

### 2.3 Stability Measurements at the Initial Time Point

All of the oxaliplatin formulations at the initial time point were clear, colourless solutions with no visible particles present in solution. The appearance of the solutions are set out in Table 7. Measurements of the pH results of the formulations are also shown in Table 7.

**Table 7 Test Results for pH and Appearance of Oxaliplatin Solutions of example 2 at Initial Time Point**

| **Formulation** | **pH initial** | **Appearance** |
|---|---|---|
| Control | 5.77 | N |
| A3.5 | 3.56 | N |
| A4.0 | 3.96 | N |
| A5.0 | 5.22 | N |
| A7.0 | 7.44 | N |
| A8.5 | 8.47 | N |
| B4.0 | 4.08 | N |
| 87,0 | 7.27 | N |
| C4.0 | 3.99 | N |
| C7.0 | 6.91 | N |
| D4.0 | 3.97 | N |
| D7.0 | 7.37 | N |
| E4.0 | 3.99 | N |
| E7.0 | 7.4 | N |
| Oxalic acid | 2.94 | N |

| | | |
|---|---|---|
| N = a clear, colourless solution with no visible particles present in solution | | |

### 2.4 Stability Measurements

The formulations were then stored at 25°C and 40°C.

The appearance of the formulations was assessed at the initial, 4 week and 8 week time points. Each formulation remained clear and colourless.

The pH of the formulations was measured at the initial, 4 week and 12 week time points for 25°C as shown in Table 8 and for 40°C as shown in Table 9.

**Table 8 Test Results for pH of Oxaliplatin Solutions of Example 2 at 25°C**

| Formulation | pH initial | pH 4wks | pH 12wks |
|---|---|---|---|
| Control | 5.77 | 5.80 | 5.73 |
| A3.5 | 3.56 | 3.54 | n/t |
| A4 | 3.96 | 3.99 | 3.89 |
| A5 | 5.22 | 5.03 | 5.15 |
| A7 | 7.44 | 5.54 | 5.54 |
| A8.5 | 8.47 | 6.26 | n/t |
| B4 | 4.08 | 4.52 | n/t |
| B7 | 7.27 | 5.43 | 5.84 |
| C4 | 3.99 | 4.03 | n/t |
| C7 | 6.91 | 5.16 | 5.44 |
| D4 | 3.97 | 4.12 | n/t |
| D7 | 7.37 | 5.31 | n/t |
| E4 | 3.99 | 4.55 | 4.75 |
| E7.0 | 7.4 | 5.61 | 6.15 |
| Oxalic acid | 2.94 | 3.42 | 3.31 |

**Table 9 Test Results for pH of Oxaliplatin Solutions of Example 2 at 40°C**

| Formulation | pH initial | pH 4wks | pH 12wks |
|---|---|---|---|
| Control | 5.77 | 5.79 | 3.30 |
| A3.5 | 3.56 | 3.57 | n/t |
| A4 | 3.96 | 3.93 | 3.81 |
| A5 | 5.22 | 5.06 | 5.01 |
| A7 | 7.44 | 5.43 | 5.64 |
| A8.5 | 8.47 | 6.17 | n/t |
| B4 | 4.08 | 4.29 | n/t |
| B7 | 7.27 | 5.61 | 5.95 |
| C4 | 3.99 | 4.17 | n/t |
| C7 | 6.91 | 5.25 | 5.43 |
| D4 | 3.97 | 4.14 | n/t |
| D7 | 7.37 | 5.36 | n/t |
| E4 | 3.99 | 4.24 | 4.89 |
| E7 | 7.4 | 4.23 | 6.10 |
| Oxalic acid | 2.94 | 3.25 | 3.32 |

### 2.4.1 Potency assay

Formulations A4, A5, A7, B7, C7, E4, E7, Oxalic and the Control were maintained at 25°C and 40°C and were assayed for potency by HPLC after 12 weeks. Table 10 reports the impurity profile determined from the potency assay for 25°C. Table 11 reports the impurity profile determined from the potency assay for 40°C.

**Table 10 Impurity profile from the potency assay for certain oxaliplatin formulations of Example 2 at 12 weeks time point at 25°C**

| Impurity | Control | A4 | A5 | A7 | B7 | C7 | E4 | E7 | Oxalic |
|---|---|---|---|---|---|---|---|---|---|
| Total of unknown impurities | 0.10 | 0.05 | 0.05 | 0.07 | 0.08 | 0.17 | 0.10 | 0.11 | 0.15 |
| R,S-Oxaliplatin | 0.01 | 0.01 | 0.01 | 0.01 | 0.01 | 0.01 | 0.01 | 0.01 | 0.01 |
| Oxaliplatin | 99.87 | 99.89 | 99.89 | 99.89 | 99.88 | 99.72 | 99.86 | 99.85 | 99.76 |
| Impurity C | 0.01 | 0.01 | 0.01 | 0.01 | 0.01 | 0.01 | 0.01 | 0.01 | 0.02 |
| Total impurity (%) | 0.12 | 0.07 | 0.07 | 0.09 | 0.1 | 0.19 | 0.12 | 0.13 | 0.18 |

**Table 11 Impurity profile from the potency assay for certain oxaliplatin formulations of Example 2 at 12 weeks time point at 40°C**

| Impurity | Control | A4.0 | A5.0 | A7.0 | B7.0 | C7.0 | E4.0 | E7.0 | Oxalic acid |
|---|---|---|---|---|---|---|---|---|---|
| Total of unknown impurities | 0.15 | 0.18 | 0.13 | 0.14 | 0.08 | 0.53 | 0.07 | 0.08 | 0.15 |
| R,S-Oxaliplatin | 0.01 | 0.01 | 0.01 | 0.01 | 0.01 | 0.02 | 0.01 | 0.01 | 0.01 |
| Oxaliplatin | 99.67 | 99.66 | 99.74 | 99.73 | 99.81 | 99.27 | 99.77 | 99.77 | 99.66 |
| Impurity C | 0.02 | 0.02 | 0.02 | 0.01 | 0.01 | 0.02 | 0.01 | 0.01 | 0.02 |
| Total impurity (%) | 0.18 | 0.21 | 0.15 | 0.16 | 0.10 | 0.57 | 0.09 | 0.10 | 0.18 |

### 2.4.2 Impurity B Assay

The level of Impurity B of the formulations maintained at 25°C was assayed by HPLC after 12 weeks for A4, A5, A7, B7, C7, E4, E7, Oxalic and the Control. Table 12 reports the impurity profile determined from that impurity B assay for 25°C. The level of impurity B of the formulations maintained at 40°C was assayed by HPLC after 8 weeks. Table 13 reports the impurity profile determined from that impurity B assay.

**Table 12 The levels of impurity B and other unknown impurities from impurity B assay in certain formulations of Example 2 at 12 weeks time point at 25°C**

| Impurity | Control | A4 | A5 | A7 | B7 | C7 | E4 | E7 | Oxalic |
|---|---|---|---|---|---|---|---|---|---|
| Total of unknown impurity | 0.07 | 0.05 | 0.06 | 0.06 | 0.04 | 0.06 | 0.02 | 0.06 | 0.06 |
| **Imp B** | 0.24 | 0.29 | 0.12 | 0.10 | 0.13 | 0.06 | 0.21 | 0.18 | 0.38 |
| **Dimer** | 0.10 | Nd | Nd | Nd | 0.02 | Nd | Nd | 0.05 | Nd |
| Total impurity (%) | 0.41 | 0.34 | 0.18 | 0.16 | 0.19 | 0.12 | 0.23 | 0.29 | 0.44 |

**Table 13 The levels of impurity B and other unknown impurities from impurity B assay in certain formulations of example 2 at the 8 weeks time point at 40°C**

| Impurity | Control | A4 | A5 | A7 | B7 | C7 | E4 | E7 | Oxalic |
|---|---|---|---|---|---|---|---|---|---|
| Total of unknown impurities | 0.15 | 0.06 | 0.09 | 0.10 | 0.09 | 0.11 | 0.06 | 0.07 | 0.05 |
| Imp B | 0.26 | 0.24 | 0.09 | 0.09 | 0.11 | 0.06 | 0.19 | 0.19 | 0.36 |
| Dimer | 0.17 | 0.01 | Nd | Nd | 0.03 | 0.01 | Nd | Nd | Nd |
| Total impurity (%) | 0.58 | 0.31 | 0.18 | 0.19 | 0.23 | 0.18 | 0.25 | 0.26 | 0.41 |

### 2.5 Stability Measurements at 9 months

Formulations A4.0, A5.0, A7.0, E4.0 and E7.0 were stored at 25°C and 40°C for 9 months and then analysed for pH and impurities.

### 2.5.1 Results and Discussion

### 2.5.1.1 Appearance Results

Appearance was clear, colourless with no visible particulate matter present in most of the formulations (Table 14).

**Table 14 Appearance of oxaliplatin solutions at different time point at 25°C**

| Formations | Appearance 9 months (25°C) | Appearance 9 months (40°C) |
|---|---|---|
| Control | N | N⁺ |
| A4 | N⁺ | N⁺⁺ |
| A5 | N | N |
| A7 | N/t | N |
| E4 | N | N |
| E7 | N | N |

| | | |
|---|---|---|
| N = a clear, colourless solution with no particulate matter present in solution N⁺ = a clear, colourless solution with few particles present in solution N⁺⁺ = a clear, colourless solution with some black particles present in solution N/t = not tested | | |

### 2.5.1.2 Impurity B Assay

Levels of impurity B and Dimer in formulations Control, A4, A5, E4 and E7 at 9 months for both 25°C and 40°C were assessed using HPLC. The results are shown in Table 15 and 16, respectively.

From the assay, the formulations A4, A5, E4 and E7 contained less total impurity than control at 25°C. At 40°C, formulations A4, A5 and E4 contained less total impurity than the control. In all cases the Dimer impurity was suppressed relative to the Control and indeed was not detected in formulations A5, A7 and E4.

**Table 15 The % of impurity B and other unknown impurities from impurity B assay in certain formulations of Example 2 at 25°C for 9 months**

| Impurities | Control | A4 | A5 | E4 | E7 |
|---|---|---|---|---|---|
| Total of unknown impurities | 0.07 | 0.05 | 0.06 | 0.03 | 0.10 |
| Imp B | 0.22 | 0.27 | 0.11 | 0.18 | 0.15 |
| dimer | 0.15 | ND | ND | ND | 0.06 |
| Total impurity | 0.44 | 0.32 | 0.17 | 0.21 | 0.31 |

| | | | | | |
|---|---|---|---|---|---|
| ND = not detected. | | | | | |

**Table 16 The % of impurity B and other unknown impurities from impurity B assay in certain formulations of Example 2 at 9 months time point at 40°C**

| Impurities | Control | A4 | A5 | A7 | E4 | E7 |
|---|---|---|---|---|---|---|
| Total of unknown impurities | 0.22 | 0.20 | 0.31 | 0.42 | 0.22 | 0.46 |
| Imp B | 0.26 | 0.21 | 0.09 | 0.08 | 0.23 | 0.12 |
| dimer | 0.14 | ND | ND | ND | ND | 0.04 |
| Total impurity | 0.62 | 0.41 | 0.40 | 0.50 | 0.45 | 0.62 |

| | | | | | | |
|---|---|---|---|---|---|---|
| ND = not detected | | | | | | |

### 2.6 Summary

The screening study indicated that tartaric acid is a suitable stabilising agent for oxaliplatin at a range of concentrations. In terms of the ability of tartaric acid to stabilise the oxaliplatin, concentrations of 0.2 mM and 0.3 mM (formulations E and A respectively) are preferred, although formulations at 0.6 mM (formulations B) also demonstrated some stabilising effect.

### Example 3

As shown in Example 1, a formulation screening study showed that the presence of tartaric acid in an oxaliplatin solution can suppress the formation of impurities relative to a control solution and thereby stabilise the formulation. The studies discussed in Example 2 indicated that the total concentration of tartaric acid in the formulation was of importance in providing a stabilising effect. The possibility existed that other carboxylic acids may have the same stabilising effect. This study involved the screening of a range of carboxylic acids (other than tartaric acid) at a set concentration (0.3 mM) in oxaliplatin solutions. The formulations were placed at 40°C for 5 weeks and then evaluated for stability. The performance of the formulations were compared to a solution of oxaliplatin containing tartaric acid at 0.3 mM.

### 3.1 Experimental

The following acids were used in the study:

| | |
|---|---|
| Tartaric acid | Maleic acid |
| Lactic acid | D-Saccharic acid |
| Citric add anhydrous | Succinic acid |
| Malonic acid | Oxalic acid |
| Malic acid | |

### 3.1.1 Procedure for making 1% acid solutions

Each acid was weighed separately into a 100mL volumetric flask. The solutions were made up to final volume after dissolving the acid completely.

### 3.1.2 Preparation of oxaliplatin formulations

About 80% mL of the desired amount of WFI was added into a clean glass beaker and heated the WFI to 50°C -55°C, while stirring and flushing with nitrogen. The oxaliplatin was then added to the beaker and mixed until clear solution was obtained (about 50 minutes was needed to achieve complete dissolution of oxaliplatin). The solution was then made up to volume with WFI. The bulk solution was divided into 100 mL quantities and the required amount of acid solution was added to each 100 mL quantity according to Table 20. Each formulation was then flushed with nitrogen until the dissolved oxygen content in solution was below 0.05 ppm.

### 3.1.1.3 Filling and capping

Each formulation was filtered using a 0.2 µm syringe filter. Then 5 mL of the solution was placed into a 10 mL vial, and capped and sealed for each formulation.

### 3.1.2 Formulation Details

Tables 19, 20 and 21 show the formulation details and quantities of oxaliplatin and excipients needed for each formulation.

**Table 19 Formulation details for the oxaliplatin solutions of Example 3 (unit formula)**

| Formulation ID | Tartaric acid | Lactic acid | Citric acid | Malonic acid | Malic acid | Maleic acid | Saccharic acid | Succinic acid | Oxalic acid |
|---|---|---|---|---|---|---|---|---|---|
| Oxaliplatin (mg) | 5 mg | 5mg | 5 mg | 5 mg | 5 mg | 5 mg | 5 mg | 5 mg | 5mg |
| Acid in mg | 0.045 | 0.027 | 0.063 | 0.031 | 0.040 | 0.035 | 0.074 | 0.035 | 0.022 |
| Acid in molar concentration (mM) | 0.3 | 0.3 | 0.3 | 0.3 | 0.3 | 0.3 | 0.3 | 0.3 | 0.3 |
| Molecular weight | 150.09 | 90.08 | 210.14 | 104 | 134.09 | 116.07 | 248.02 | 118.09 | 72 |
| WF1 qs (mL) | 1mL | 1mL | 1mL | 1mL | 1mL | 1mL | 1mL | 1 mL | 1mL |

**Table 20 Quantity required for the oxaliplatin solutions of Example 3**

| Formulation ID | Tartaric acid | Lactic acid | Citric acid | Malonic acid | Malic add | Maleic acid | Saccharic acid | Succinic acid | Oxalic acid |
|---|---|---|---|---|---|---|---|---|---|
| Oxaliplatin (mg) | 500 mg | 500 mg | 500 mg | 500 mg | 500 mg | 500 mg | 500 mg | 500 mg | 500 mg |
| Acid (mg) | 4.50 | 2.70 | 6.30 | 3.12 | 4.02 | 3.48 | 7.44 | 3.54 | 2.16 |
| 1% acid in uL | 450.0 | 270.0 | 630.4 | 312.0 | 402.3 | 348.2 | 744.1 | 354.3 | 216.0 |
| WFI qs (mL) | 100 mL | 100 mL | 100 mL | 100 mL | 100 mL | 100mL | 100mL | 100 mL | 100 mL |

**Table 21 Actual quantity added for the oxaliplatin solutions of Example 3**

| Formulation ID | Tartaric acid | Lactic acid | Citric acid | malonic | Malic acid | Maleic | Saccharic acid | Succinic | Oxalic acid |
|---|---|---|---|---|---|---|---|---|---|
| Oxaliplatin bulk solution | 100 mL | 100 mL | 100 mL | 100 mL | 100 mL | 100 mL | 100 mL | 100 mL | 100 mL |
| (5 mg/mL) | | | | | | | | | |
| 1% acid in uL | 450.0 | 355 | 630 | 315 | 405 | 350 | *7.45 mg | 355 | 216.0 |
| WFI qs (mL) | 100 mL | 100 mL | 100 mL | 100 mL | 100 mL | 100mL | 100mL | 100 mL | 100 mL |

| | | | | | | | | | |
|---|---|---|---|---|---|---|---|---|---|
| * Saccharic acid was added as powder in 100 mL bulk solution to give 0.3 mM acid concentration. | | | | | | | | | |

### 3.2 Stability evaluation at 5 weeks at 40°C

The oxaliplatin solutions containing the various carboxylic acids were formulated and place at 40°C at 75%for stability evaluation. The formulations was evaluated at the five weeks time points and the results are reported below.

### 3.2.1 Results and Discussion

### 3.2.1.1 pH and Appearance

The pH and appearance results for the formulations of Example 3 are shown in Table 22.

**Table 22 Appearance and pH of oxaliplatin formulations of Example 3 for 5 weeks**

| **Formulations ID** | **Initial** | | **5 weeks** | |
|---|---|---|---|---|
| | **Appearance** | **pH** | **Appearance 40°C** | **pH 40°C** |
| Oxaliplatin/tartaric | N/t | 3.65 | N | 3.70 |
| Oxaliplatin/lactic | N/t | 3.94 | N⁺ | 4.01 |
| Oxaliplatin/citric | N/t | 3.65 | N | 3.61 |
| Oxaliplatin/malonic | N/t | 3.69 | N | 3.64 |
| Oxaliplatin/malic | N/t | 3.81 | N | 3.91 |
| Oxaliplatin/maleic | N/t | 3.64 | N | 3.68 |
| Oxaliplatin/saccharic | N/t | 5.34 | N⁺⁺ | 4.75 |
| Oxaliplatin/succinic | N/t | 4.05 | N | 4.09 |
| Oxaliplatin/oxalic | N/t | 3.76 | N | 3.89 |

| | | | | |
|---|---|---|---|---|
| *N = clear colourless solution with no visible particles.* *N' = clear colorless solution with few black particles.* *N⁺⁺ = clear colorless solution with many white particles.* *N*/*t=not tested.* | | | | |

### 3.2.1.2 Potency and Impurity Results

The potency of the formulations of Example 3 was measured by HPLC after 5 weeks at 40°C (Table 23). Each of the formulations maintained a potency above 95%.

**Table 23 Potency results at initial and 5 weeks for the oxaliplatin solutions of Example 3 at 40°C.**

| **Formulation ID** | **Initial (mg/mL)** | **5 weeks (mg/mL)** | **% of initial** |
|---|---|---|---|
| Oxaliplatin/tartaric | 5.19 | 5.17 | 99.6 |
| Oxaliplatin/lactic | 5.30 | 5.18 | 97.7 |
| Oxaliplatin/citric | 5.19 | 5.15 | 99.2 |
| Oxallplatin/malonic | 5.21 | 5.18 | 99.4 |
| Oxaliplatin/malic | 5.21 | 5.15 | 98.9 |
| Oxaliplatin/maleic | 5.20 | 5.06 | 97.3 |
| Oxaliplatin/saccharic | 5.22 | 5.01 | 96.0 |
| Oxaliplatin/succinic | 5.22 | 5.17 | 99.0 |
| Oxaliplatin/oxalic | 5.24 | 5.21 | 99.4 |

The formulations of Example 3 were also assayed by HPLC for the presence of impurity B and Dimer after 5 weeks at 40°C. Table 24 reports the results of this assay.

**Table 24 Test results at 5 weeks at 40°C of the formulations of Example 3 for impurity B and the dimer impurity.**

| **Formulation ID** | **% Impurity B** | **% Dimer** |
|---|---|---|
| Oxaliplatin/tartaric | 0.36 | ND |
| Oxaliplatin/lactic | 0.34 | ND |
| Oxaliplatin/citric | 0.26 | ND |
| Oxaliplatin/malonic | 0.27 | ND |
| Oxaliplatin/malic | 0.31 | ND |
| Oxaliplatin/malcic | 0.18 | ND |
| Oxaliplatin/saccharic | 0.28 | ND |
| Oxaliplatin/succinic | 0.27 | ND |
| Oxaliplatin/oxalic | 0.17 | ND |

| | | |
|---|---|---|
| **ND: None detected* | | |

Table25 summarises the impurity profile obtained from the assay for potency by HPLC which was carried out by IIPLC at the 5 week time points.

**Table 25 Impurity profile from potency assay for oxaliplatin solution formulations of Example 3 at the 5 weeks time point at 40°C.**

| Impurity | Tartaric acid | Lactic acid | Citric acid | Malonic acid | Malic acid | Maleic acid | Saccharic acid | Succinic acid | Oxalic acid |
|---|---|---|---|---|---|---|---|---|---|
| Total of unknown impurities | 0.01 | 0.09 | 0.31 | 5.93 | 0.0 | 0.33 | 0.42 | 0.03 | 027 |
| R,S-Oxaliplatin | 0.01 | 0.01 | 0.01 | 0.02 | 0.01 | 0.01 | 0.02 | 0.01 | 0.01 |
| Oxaliplatin | 99.91 | 99.71 | 99.59 | 93.20 | 99.89 | 99.58 | 99.36 | 99.90 | 99.49 |
| Impurity C | 0.01 | 0.03 | 0.02 | 0.20 | 0.02 | 0.02 | 0.05 | 0.01 | 0.05 |
| Total impurity from | 0.03 | 0.13 | 0.34 | 6.15 | 0.03 | 0.36 | 0.49 | 0.05 | 0.33 |
| potency assay (%) | | | | | | | | | |

The total impurity levels for the Example 3 formulations from the HPLC assay for potency and the HPLC assay for Impurity B after 5 weeks at 40°C are shown in Table 26.

**Table 26 Total impurity for oxaliplatin solution formulations of example 3 at 5 weeks time point at 40°C**

| Impurity | Tartaric acid | Lactic acid | Citric acid | Malonic acid | Malic acid | Maleic acid | Saccharic acid | Succinic acid | Oxalic acid |
|---|---|---|---|---|---|---|---|---|---|
| Total impurity from potency assay (%) | 0.03 | 0.13 | 0.34 | 6.15 | 0.03 | 0.36 | 0.49 | 0.05 | 0.33 |
| Impurity B(%) | 0.36 | 0.27 | 0.17 | 0.18 | 0.31 | 0.26 | 0.28 | 0.34 | 0.27 |
| Dimer (%) | ND | ND | ND | ND | ND | ND | ND | ND | ND |
| Total impurity (%) | 0.39 | 0.40 | 0.51 | 6.33 | 0.34 | 0.62 | 0.77 | 0.39 | 0.60 |

### 3.3 Summary

Formulations containing malic and succinic acid showed impurity levels which were comparable to the tartaric acid formulation. Contrary to the teaching of the prior art, the malonic acid formulation contained a surprisingly and unacceptably high level of impurities relative to the tartaric acid formulation. Solutions containing citric, maleic and saccharic acids also showed reasonably low levels of impurity and would be considered to be suitable buffering agents for oxaliplatin. The stabilisers of the prior art, oxalic acid and lactic acid, also displayed reasonably low levels of impurity which is unsurprising in the case of oxalic acid due to the operation of Le Chatelier's principle.

### Example 4

The following formulation was prepared for the purpose of regulatory testing:
Oxaliplatin 5mg
Tartaric acid 0.03 mg
NaOH (adjust to pH of approximately 5)
WFT qs 1 mL

The pH is adjusted to pH 5 with a range of from 4.7 to 5.5 using NaOH. The concentration of tartaric acid is about 0.2 mM.

Throughout this specification, the word "comprise", or variations such as "comprises" or "comprising" will be understood to imply the inclusion of a stated element, integer or step, or groups of elements, integers or steps, but not the exclusion of any other element, integer or step, or groups of elements, integers or steps.

Any discussion of documents, acts, materials, devices, articles or the like which has been included in the present specification is solely for the purpose of providing a context for the present invention. It is not to be taken as an admission that any or all of these matters form part of the prior art base or were common general knowledge in the field relevant to the present invention as it existed in Australia before the priority date of each claim of the application.

It will be appreciated by persons skilled in the art that numerous variations and/or modifications may be made to the invention as shown in the specific embodiments without departing from the spirit or scope of the invention as broadly described. The present embodiments are, therefore, to be considered in all respects as illustrative and not restrictive.

## Claims

1. A pharmaceutical liquid formulation of oxaliplatin for parenteral administration, said formulation comprising
(i) oxaliplatin,
(ii) water; and
(iii) an additive selected from the group consisting of tartaric acid, a salt of tartaric acid, a pharmaceutically acceptable derivative of tartaric acid and mixtures thereof; wherein the additive is at a concentration of at least 0.01 mM.

2. A formulation according to claim 1 wherein the concentration of the additive is from about 0.01 mM to about 2.0 mM.

3. A formulation according to claim 1 or claim 2 wherein the concentration of the additive is from about 0.1 mM to about 1.0 mM.

4. A formulation according to any one of claims 1 to 3 wherein the concentration of the additive is from about 0.2 mM to about 0.6 mM.

5. A formulation according to any one of claims 1 to 4 wherein the additive comprises a salt of tartaric acid and wherein the salt is a sodium salt.

6. A formulation according to any one of claims 1 to5 wherein the concentration of oxaliplatin is up to about 15 mg/ml.

7. A formulation according to any one of claims 1 to 6 wherein the pH of the formulation is in the range of from about 3 to about 7.

8. A pharmaceutical liquid formulation of oxaliplatin for parenteral administration, said formulation comprising
(i) about 5 mg/ml of oxaliplatin,
(ii) water, and
(iii) an additive consisting of tartaric acid and the sodium salt of tartaric acid, wherein the concentration of the additive is about 0.2 mM and wherein the pH of the solution is from about 4.7 to about 5.5.

9. A pharmaceutical formulation according to any one of claims 1 to 8 for use in the treatment of a cancer.

10. A method for preparing a pharmaceutical formulation according to any one of claims 1 to 8, the method comprising the steps of:
(i) dissolving oxaliplatin in water to form a solution;
(ii) dissolving in the solution an additive selected from the group consisting of a tartaric acid, a salt of tartaric acid, a pharmaceutically acceptable derivative of a pharmaceutically acceptable tartaric acid and mixtures thereof;
(iii) optionally, adjusting the pH of the solution with a pharmaceutically acceptable base.

11. A method according to claim 10 wherein the pharmaceutically acceptable base is sodium hydroxide.

12. A pharmaceutical liquid formulation of oxaliplatin for parenteral administration, said formulation comprising
(i) oxaliplatin;
(ii) water; and
(iii) an acid
wherein the acid is stabilising and is not malonic acid, lactic acid or oxalic acid.

13. A pharmaceutical liquid formulation of oxaliplatin for parenteral administration, said formulation comprising
(i) oxaliplatin;
(ii) water; and
(iii) an acid comprising at least 4 carbon atoms.

14. A pharmaceutical liquid formulation of oxaliplatin for parenteral administration, said formulation comprising
(i) oxaliplatin,
(ii) water; and
(iii) an additive selected from the group consisting of a pharmaceutically acceptable carboxylic acid, a salt of a pharmaceutically acceptable carboxylic acid, a pharmaceutically acceptable derivative of a pharmaceutically acceptable carboxylic acid and mixtures thereof;
wherein the additive is at a concentration of at least 0.01 mM and wherein the acid is not malonic acid, lactic acid or oxalic acid.

15. A pharmaceutical liquid formulation of oxaliplatin for parenteral administration, said formulation comprising
(i) oxaliplatin,
(ii) water; and
(iii) an additive selected from the group consisting of a pharmaceutically acceptable carboxylic acid, a salt of a pharmaceutically acceptable carboxylic acid, a pharmaceutically acceptable derivative of a pharmaceutically acceptable carboxylic acid and mixtures thereof;
wherein the additive is at a concentration of at least 0.01 mM and wherein the carboxylic acid is of the formula:
HO2C[C(R1)(R2)]nCO2H
wherein n = 2 to 6; and R1 and R2 are each independently selected from the group consisting of H, OH, CO2H, halo and methyl.
